# EUROPEAN PATENT SPECIFICATION

(11) **EP 4 427 705 B1**
(45) Date of publication and mention of the grant of the patent: **04.11.2026**
(21) Application number: 22901859.3
(22) Date of filing: 02.12.2022
(51) Int. Cl.: A61B 90/00, A61F 2/14, A61F 9/007, A61F 9/00, A61B 34/00, A61L 27/36, A61B 17/00

(54) **CORNEAL ENDOTHELIAL TISSUE TRANSPLANTATION SYSTEM**
TRANSPLANTATIONSSYSTEM FÜR HORNHAUTENDOTHELGEWEBE
SYSTÈME DE TRANSPLANTATION DE TISSU ENDOTHÉLIAL CORNÉEN

(30) Priority: 03.12.2021 KR 20210172039
(43) Date of publication of application: 11.09.2024
(73) Proprietor: The Asan Foundation, Seoul 05505 (KR); University Of Ulsan Foundation For Industry Cooperation, Ulsan 44610 (KR)
(72) Inventor: LEE, Hun, Seoul 06503 (KR); HWANG, Chang Mo, Seoul 05505 (KR)
(74) Representative: AWA Sweden AB
(86) International application number: PCT/KR2022/019448
(87) International publication number: WO 2023/101501

(56) References cited:
- WO-A1-2013/076723
- WO-A1-2013/076723
- WO-A1-2014/124073
- JP-A- 2017 503 608
- KR-A- 20180 017 271
- US-A1- 2012 226 107
- US-B1- 6 306 075
- US-B1- 6 306 075

## Description

### Technical Field

The present disclosure relates to a corneal endothelial tissue transplantation system.

### Background Art

An anterior chamber of an eyeball refers to a space in the eyeball surrounded by a cornea, an iris, and a lens, and corneal endothelial tissue transplantation is performed in the anterior chamber of the eyeball. Specifically, corneal endothelial tissue transplantation is performed by inserting corneal endothelial tissue into an anterior chamber of an eyeball, moving the inserted corneal endothelial tissue, and then transplanting the inserted corneal endothelial tissue to a back surface of a cornea.

Meanwhile, an anterior chamber of an eyeball has only a diameter of about 11 mm to about 13 mm and a depth of 3 mm to 4 mm and thus is very narrow. Therefore, there is a problem that, after a corneal endothelial tissue is inserted into an anterior chamber, it is very difficult to insert a tool for move the corneal endothelial tissue to a desired position or to operate the inserted tool.

In particular, since corneal endothelial tissue is very thin tissue with a thickness of about 10 µm, there is also a problem that the tissue may be damaged when the corneal endothelial tissue is handled by using a sharp tool such as forceps.

Due to these problems, corneal endothelial tissue transplantation requires very sophisticated and high-level surgical techniques, and thus the corneal endothelial tissue transplantation may only be performed by medical staff skilled in the art. However, in reality, skilled medical staff who may perform corneal endothelial tissue transplantation are very rare, which makes it difficult for patients in need of corneal endothelial tissue transplantation to receive surgery in a timely manner.

WO 2013/076723 discloses a corneal endothelial tissue transplantation system comprising a tissue carrier and a magnetic field generation module.

US 6 306 075 discloses a system for corneal re-shaping comprising magnetic field emitters.

WO 2014/124073 discloses a magnetic operating microscope which may comprise a ring shaped magnet. According to the disclosure, the electromagnet may be in the form of a coil.

Accordingly, there is a need to develop a means for transplanting corneal endothelial tissue, which is capable of easily and precisely moving corneal endothelial tissue inserted into an anterior chamber and furthermore is capable of ensuring the safety of an eyeball of a patient.

### Detailed Description of the disclosure

### Technical Problem

The present disclosure is directed to providing a corneal endothelial tissue transplantation system for easily and precisely moving corneal endothelial tissue inserted into an anterior chamber of an eyeball.

The present disclosure is also directed to providing a corneal endothelial tissue transplantation system which is capable of minimizing damage to corneal endothelial tissue.

However, the objects are only for illustration and should not be used to limit the scope of the present disclosure.

### Technical Solution to Problem

The invention is defined in the claims. In order to achieve the above object, a corneal endothelial tissue transplantation system according to the appended claims includes a tissue carrier to which cultured corneal endothelial tissue is attached and inserted into an anterior chamber of an eyeball, a magnetic field generation module including at least one magnetic field generator which generates a magnetic field for moving the tissue carrier, and a support plate which supports the magnetic field generator, and a user input module which receives an input signal for controlling the magnetic field generator from a user.

### Advantageous Effects of the Disclosure

There may be provided a corneal endothelial tissue transplantation system for easily and precisely moving corneal endothelial tissue inserted into an anterior chamber of an eyeball.

In addition, there may be provided a corneal endothelial tissue transplantation system which is capable of minimizing damage to corneal endothelial tissue.

However, the effects are only for illustration, and the effects of the present disclosure is not limited thereto.

### Brief Description of Drawings

FIG. 1 is a schematic view for describing corneal endothelial tissue transplantation.
FIG. 2 is a schematic block diagram illustrating a corneal endothelial tissue transplantation system according to an embodiment of the present disclosure.
FIG. 3 is a schematic view illustrating an embodiment of a tissue carrier of FIG. 2.
FIG. 4 is a schematic view illustrating an embodiment of a magnetic field generation module of FIG. 2.
FIG. 5 is a schematic view illustrating a magnetic field generator of FIG. 4 viewed from the side.
FIG. 6 is an enlarged view of portion A of FIG. 4.
FIG. 7 is a schematic view illustrating a process of attaching corneal endothelial tissue to a cornea.

### Best Mode

In order to achieve the above object, a corneal endothelial tissue transplantation system (100) is provided. The system is comprising:
a tissue carrier (110) configured to be attached to cultured corneal endothelial tissue and configured to be inserted into an anterior chamber of an eyeball,
a magnetic field generation module (120) comprising a plurality of magnetic field generators (121) which generate a magnetic field for moving the tissue carrier (110), and a support plate (122) which supports the magnetic field generator (121);
a user input module (140) which receives an input signal for controlling the magnetic field generator (121) from a user.

The support plate includes a surgical port (1221) formed through the support plate (122) configured to observe the inserted tissue carrier (110); and wherein the plurality of magnetic field generators (121) are disposed on one surface of the support plate (122) along a circumference of the surgical port (1221) such that the generated magnetic field is concentrated in a direction toward the surgical port. The tissue carrier comprises a wire (111) disposed along at least a portion of a circumference of the tissue carrier (110), and the wire includes a magnetic material.

In addition, the tissue carrier may be formed in a plate shape.

In addition, the tissue carrier may be inserted into the anterior chamber of the eyeball in a state in which at least a portion thereof is folded, and after the tissue carrier is inserted into the anterior chamber of the eyeball, the tissue carrier may be unfolded by the magnetic field generation module.

In addition, the support plate includes a surgical port for observing the inserted tissue carrier.

In addition, the magnetic field generator is disposed along a circumference of the surgical port.

In addition, the magnetic field generator may be disposed to be inclined in a direction toward the tissue carrier.

In addition, the magnetic field generator may include a core around which a coil is wound, and the core may include a curved portion bent at a certain angle in a direction toward the tissue carrier.

In addition, the corneal endothelial tissue transplantation system may further include air supply module which supplies air to the inserted tissue carrier.

### Mode for Disclosure

Since the present disclosure can apply various transformations and have various embodiments, specific embodiments will be illustrated in the drawings and described in detail in the detailed description. Effects and features of the present disclosure, and methods for achieving them will become clear with reference to the embodiments described below in detail together with the drawings. However, the present disclosure is not limited to the embodiments disclosed below and may be implemented in various forms.

Hereinafter embodiments of the present disclosure will be described in detail with reference to the accompanying drawings, wherein like reference numerals refer to the same or corresponding components throughout the drawings, and a redundant description thereof will be omitted.

In the following embodiments, the terms first, second, and the like do not have limited meaning but are used for the purpose of distinguishing one component from another component.

In the following embodiments, the expressions used in the singular such as "a," "an," and "the" are intended to include the plural forms as well, unless the context clearly indicates otherwise.

In the following embodiments, it will be understood that the terms such as "including," "comprising," and "having" specify the presence of stated features or components, but do not preclude the presence or addition of one or more other features or components.

In the drawings, components may be exaggerated or reduced in size for convenience of description. For example, the sizes and thicknesses of the respective components shown in the drawings are arbitrarily shown for convenience of description, and thus one or more embodiments are not necessarily limited thereto.

In the following embodiments, an X-axis, a Y-axis, and a Z-axis are not limited to three axes in a Cartesian coordinate system, but may be interpreted in a broad sense including the three axes. For example, the X-axis, Y-axis, and Z-axis may be orthogonal to each other, but may also refer to different directions that are not orthogonal to each other.

When a certain embodiment may be implemented differently, a specific process order may be performed differently from the described order. For example, two consecutively described processes may be performed substantially at the same time or performed in an order opposite to the described order.

Hereinafter, a corneal endothelial tissue transplantation system according to the present disclosure will be described based on the above-described principles.

The corneal endothelial tissue transplantation system according to an embodiment of the present disclosure may be used for corneal endothelial tissue transplantation.

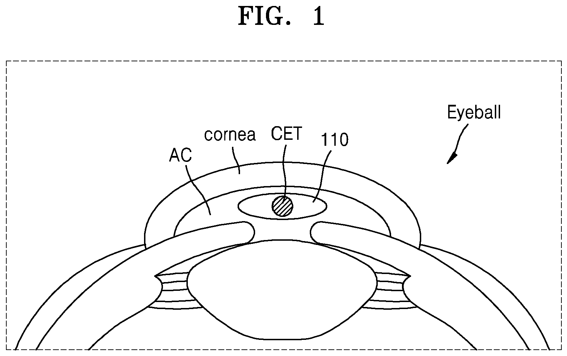

FIG. 1 is a schematic view for describing corneal endothelial tissue transplantation.

The corneal endothelial tissue transplantation refers to ophthalmic surgery in which cultured corneal endothelial tissue (hereinafter referred to as CET) is inserted into an anterior chamber (hereinafter referred to as AC) of an eyeball and then transplanted to a back surface of a cornea.

Specifically, the corneal endothelial tissue transplantation is performed by inserting the CET into the AC of the eyeball, moving the CET inside the AC of the eyeball, positioning the CET at a position suitable for transplantation, and then attaching the CET to the back surface of the cornea. In this case, the CET may be cultured in a tissue carrier 110 for transporting tissue and then inserted into the AC of the eyeball together with the tissue carrier 110. However, since the AC of the eyeball is a very narrow space, it is very difficult to insert a tool for moving the CET into the AC of the eyeball or to operate the inserted tool. In addition, since the CET is very thin microscopic tissue, there is a high risk of damage to tissue when the CET is handled by using a sharp tool such as forceps.

In order to solve such a problem, according to a corneal endothelial tissue transplantation system 100 according to the present disclosure, there may be provided a corneal endothelial tissue transplantation system 100 capable of easily and precisely manipulating CET without inserting a separate tool into an AC of an eyeball.

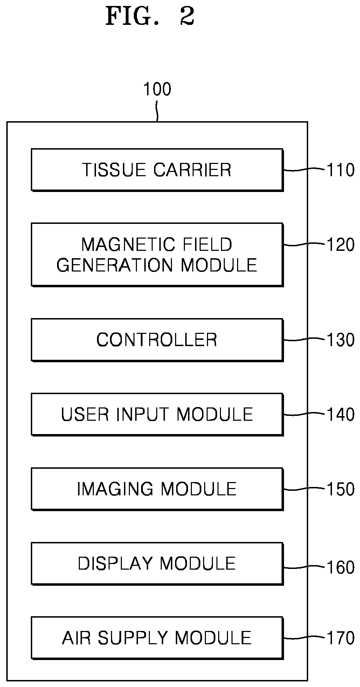

FIG. 2 is a schematic block diagram illustrating the corneal endothelial tissue transplantation system 100 according to an embodiment of the present disclosure.

Referring to FIG. 2, the corneal endothelial tissue transplantation system 100 according to an embodiment of the present disclosure may include a tissue carrier 110 to which the CET is attached and inserted into the AC of the eyeball, a magnetic field generation module 120 including at least one magnetic field generator 121 that generates a magnetic field for moving the tissue carrier 110, and a support plate 122 that supports the magnetic field generator 121, and a user input module 140 that receives a signal for controlling the magnetic field generator 121 from a user.

The tissue carrier 110 may be a means for moving the CET. For example, the CET may be cultured on the tissue carrier 110, and the tissue carrier 110 may be inserted into the AC of the eyeball in a state in which the CET is attached thereto. Accordingly, by moving the inserted tissue carrier 110, the CET may be moved together with the tissue carrier 110.

The tissue carrier 110 may be movable by a magnetic field. For example, the tissue carrier 110 may include a magnetic object, and accordingly, when a magnetic field is applied from the outside, the tissue carrier 110 may move in response to the magnetic field.

Details of the tissue carrier 110 will be described below.

The magnetic field generation module 120 is a means for generating a magnetic field to move the tissue carrier 110.

The magnetic field generation module 120 includes a plurality of the magnetic field generator 121. The magnetic field generator 121 may be a component that converts supplied electrical energy into magnetic energy. A plurality of magnetic field generators 121 is disposed, and power supplied to each magnetic field generator 121 may be controlled. Therefore, a user may change power supplied to each magnetic field generator 121 to control the intensity of a magnetic field generated by each magnetic field generator 121, thereby moving the tissue carrier 110.

Details of the magnetic field generation module 120 will be described below.

The corneal endothelial tissue transplantation system 100 according to an embodiment of the present disclosure may further include a controller 130 for controlling each component of the corneal endothelial tissue transplantation system 100.

The controller 130 may be implemented as at least one processor. When the controller 130 is implemented as a plurality of processors, at least some of the plurality of processors may be positioned at a physical separation distance. For example, the controller 130 may correspond to at least one processor. The processor may be implemented as an array of a plurality of logic gates or may be implemented as a combination of a general-purpose microprocessor and a memory in which a program executable in the microprocessor is stored. In addition, it will be understood by one of ordinary skill in the art that the processor can be implemented in other forms of hardware.

The user input module 140 is a means for receiving any type of input signal input by a user.

For example, the user input module 140 may be a typical computing input device such as a joystick, a keyboard, a mouse, or a touch screen or may be a means for recognizing a user' voice or eyeball movement, but is not limited thereto. That is, as the user input module 140, any means may be adopted as long as the means may receive any signal through an operation of a user.

When a user performs a specific operation, the user input module 140 may receive a specific signal based on the operation and may transmit the specific signal to the controller 130. Specifically, the user input module 140 may receive a specific signal through an operation of a user and may transmit the specific signal to the controller 130. The controller 130 may receive the signal transmitted by the user input module 140 and may control the magnetic field generator 121 based on the received signal.

As an alternative embodiment, when an input exceeding preset intensity is applied to the user input module 140, the controller 130 may recognize the input as a user input.

For example, when the user input module 140 is a joystick, only when a user moves the joystick in a specific direction with a force that is greater than or equal to a preset force, the controller 130 may recognize such an input as a user input, and when a force less than the preset force is applied, it may be ignored. Therefore, when a minute input such as an input due to a hand tremor of a user is applied to the joystick, such a minute input may be erased, and only when an input exceeding preset intensity is applied, the controller 130 may recognize the input to control the magnetic field generator 121 based on the input.

As an embodiment, the corneal endothelial tissue transplantation system 100 according to an embodiment of the present disclosure may further include an imaging module 150.

The imaging module 150 may be a means for photographing a surgery scene and providing the photographed surgery scene to a user through a screen. The user may perform corneal endothelial transplantation while observing the surgery scene obtained and provided through the imaging module 150 in real time.

The imaging module 150 may be a camera, a microscope, or the like, but is not limited thereto.

As an embodiment, the corneal endothelial tissue transplantation system 100 according to an embodiment of the present disclosure may further include a display module 160.

The display module 160 may be a means for displaying information related to surgery to a user.

As an example, the display module 160 may provide a surgery image obtained from the imaging module 150 to the user.

As another example, the display module 160 may display information, which is related to the intensity or direction of a magnetic field generated by each magnetic field generator 121, to the user.

However, the present disclosure is not limited thereto and the display module 160 may provide the user with various types of information related to corneal endothelial transplantation.

Accordingly, the user may perform corneal endothelial transplantation while observing an image or information provided through the display module 160 in real time.

As the display module 160, any means may be adopted as long as the means may provide the user with an image acquired from the imaging module 150 or information related to surgery.

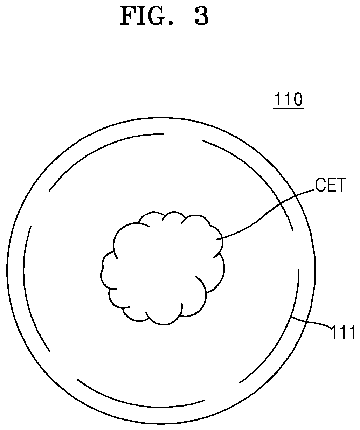

FIG. 3 is a schematic view illustrating an embodiment of the tissue carrier 110 of FIG. 2.

Referring to FIG. 3, the tissue carrier 110 may be a means for transporting a CET.

In an embodiment, the tissue carrier 110 may be formed in a plate shape to have an area. In this case, the CET may be cultured on one surface of the tissue carrier 110. That is, the cultured CET may be inserted into an AC of an eyeball of a user in a state of being attached to one surface of the tissue carrier 110.

As an alternative embodiment, the tissue carrier 110 may be formed in a plate shape having an area and may be formed in a shape that is convex in one direction. Since the human cornea is formed to have a curved surface, when the tissue carrier 110 is formed to be convex in one direction, the tissue carrier 110 may be more stably attached to the cornea together with the CET later.

As a specific example, the tissue carrier 110 may be formed in a contact lens-like shape. In this case, as the tissue carrier 110, an cornea of another animal may be used, for example, a cornea of a pig may be used, but the present disclosure is not limited thereto.

The tissue carrier 110 includes a wire 111, and the wire 111 includes a magnetic material. Therefore, when a magnetic field is applied from the outside, the wire 111 may move in response to the magnetic field, and thus the tissue carrier 110 may move.

The wire 111 is disposed along a circumference of the tissue carrier 110. Accordingly, when a magnetic field is applied in various directions, the tissue carrier 110 may easily move in response to the magnetic field.

Meanwhile, although a plurality of wires 111 are shown in FIG. 3 to be disposed along the circumference of the tissue carrier 110, the present disclosure 1 is not limited thereto, and of course, the wire 111 may be formed integrally along the circumference of the tissue carrier 110. That is, the wire 111 may be formed integrally along the circumference of the tissue carrier 110, may be provided as two wires 111, or may be provided two or more wires 111.

In an embodiment, in a state in which at least a portion of the tissue carrier 110 is folded, the tissue carrier 110 may be inserted into the AC of the eyeball. For example, the tissue carrier 110 may being inserted into the AC of the eyeball in a state of being folded in various directions and thus may be easily inserted into the AC of the eyeball.

The tissue carrier 110 may be inserted into the AC of the eyeball and then unfolded by a magnetic field. Specifically, the tissue carrier 110 may be inserted into the AC of the eyeball and then exposed to a magnetic field in various directions by the magnetic field generator 121 disposed along the circumference of the tissue carrier 110. Accordingly, a user may easily unfold a folded portion of the tissue carrier 110 by adjusting the relative intensity of various applied magnetic fields.

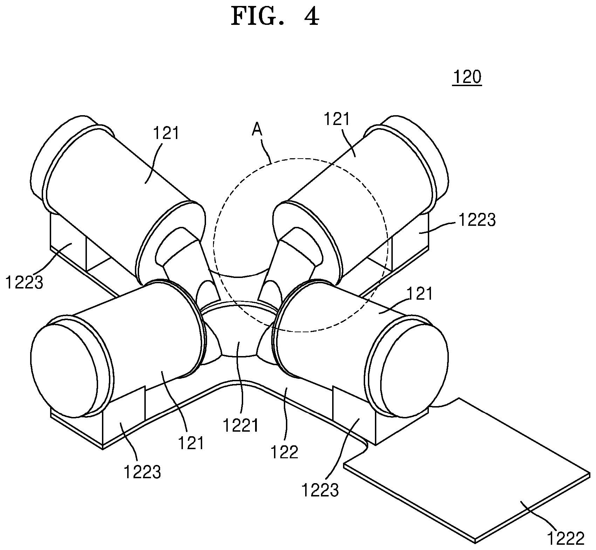

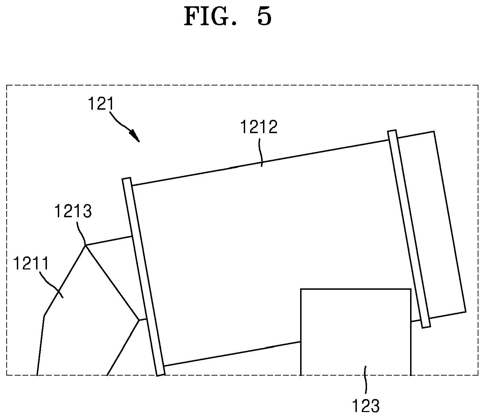

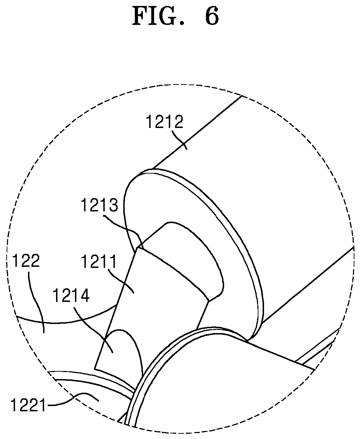

FIG.4 is a schematic view illustrating an embodiment of the magnetic field generation module 120 of FIG. 2. FIG. 5 is a schematic view illustrating the magnetic field generator 121 of FIG. 4 viewed from the side. FIG. 6 is an enlarged view of portion A of FIG. 4.

Referring to FIGS. 4 to 6, the corneal endothelial tissue transplantation system 100 according to the present disclosure may include the magnetic field generation module 120.

The magnetic field generation module 120 may generate a magnetic field to move the tissue carrier 110 inserted into an AC of an eyeball of a patient.

The magnetic field generation module 120 comprises a plurality of magnetic field generators 121 that generates a magnetic field for moving the tissue carrier 110, and the support plate 122 that supports the magnetic field generator 121.

The magnetic field generator 121 may convert supplied electrical energy into magnetic energy. To this end, the magnetic field generator 121 may include a core 1211 and a coil.

Specifically, the coil may be wound along an outer circumference surface of the core 1211, and a current supplied from a separately provided power source may flow through the coil. Accordingly, a magnetic flux may be induced on the core 1211, and thus a magnetic field may be generated.

As an embodiment, at least one magnetic field generator 121 may be provided, and more specifically, the plurality of magnetic field generators 121 may be provided. When the plurality of magnetic field generators 121 are provided, a magnetic field may be applied to the tissue carrier 110 in various directions, thereby allowing the tissue carrier 110 to move in various directions.

In a desirable embodiment, referring to FIG. 4, four magnetic field generators 121 may be disposed to face one point as a center. For example, the magnetic field generators 121 may be disposed to form an angle of 90° around one point.

Accordingly, the tissue carrier 110 disposed adjacent to a surgical port 1221 to be described below may be exposed to magnetic fields generated in at least four directions, and thus the tissue carrier 110 may be moved more precisely and efficiently.

However, the present disclosure is not limited thereto, of course, two or three magnetic field generators 121 may be provided, and five or more magnetic field generators 121 may also be provided.

The support plate 122 may be a means for supporting the magnetic field generator 121. For example, the support plate 122 may be formed in a plate shape to have an area, and the plurality of magnetic field generators 121 may be disposed on one surface of the support plate 122.

As an embodiment, the support plate 122 may be formed in a shape corresponding to an arrangement of the magnetic field generators 121.

Referring again to FIG. 4, for example, when four magnetic field generators 121 are disposed, the support plate 122 may be formed in an approximate cross shape such that the four magnetic field generators 121 are disposed. Accordingly, even when the support plate 122 is positioned adjacent to an eyeball of a patient to perform corneal endothelial transplantation, the support plate 122 may hardly block a field of view of a user or a field of view of the imaging module 150. In addition, the weight of the support plate 122 may be reduced, which may make it easier to perform corneal endothelial transplantation or to move and operate the magnetic field generation module 120.

The support plate 122 includes the surgical port 1221 for observing the tissue carrier 110. The surgical port 1221 is formed to pass through the support plate 122. That is, the surgical port 1221 may be a hole drilled in the support plate 122. Therefore, during surgery, a user may observe a surgery scene with the naked eye through the surgical port 1221. In addition, the imaging module 150 may acquire a surgery image by photographing a surgery scene through the surgical port 1221.

As a specific embodiment, referring again to FIG. 4, when the plurality of magnetic field generators 121 are disposed, the surgical port 1221 may be formed at a central portion of the plurality of magnetic field generators 121. To this end, the plurality of magnetic field generators 121 may be disposed along a circumference of the surgical port 1221, and specifically, the plurality of magnetic field generators 121 may be disposed in a direction toward the surgical port 1221.

Through such an arrangement, when a user operates the user input module 140 to move the tissue carrier 110, the magnetic field generator 121 may generate a magnetic field, and the generated magnetic field may be concentrated in the direction toward the surgical port 1221. In addition, since the plurality of magnetic field generators 121 are disposed along the circumference of the surgical port 1221, a magnetic field generated by an input of a user may move the tissue carrier 110 in a width direction of the support plate 122 in FIG. 4.

As an alternative embodiment, the support plate 122 may further include a flat portion 1222 extending from one side. The flat portion 1222 may be formed in a plate shape having a width.

As an embodiment, a circuit board may be disposed on the flat portion 1222.

Meanwhile, the flat portion 1222 may be a portion gripped by a user. For example, in order to perform corneal endothelial transplantation, while gripping the flat portion 1222, the user may move the magnetic field generation module 120 to a position adjacent to an eyeball of a patient.

In addition, while gripping the flat portion 1222, the user may move the magnetic field generation module 120 in a direction perpendicular to the width direction of the support plate 122. Accordingly, a magnetic field applied to the tissue carrier 110 may be adjusted in the direction perpendicular to the width direction of the support plate 122.

That is, assuming that the width direction of the support plate 122 corresponds to an xy plane, the user may operate the user input module 140 to perform control such that a direction of a magnetic field applied to the tissue carrier 110 is directed in x-axis and y-axis directions. By gripping the flat portion 1222, the user may move the magnetic field generation module 120 in a z-axis direction to perform control such that the direction of the magnetic field applied to the tissue carrier 110 is directed in the z-axis direction.

Referring again to FIGS. 5 and 6, the magnetic field generator 121 will be described in more detail.

As an embodiment, the magnetic field generator 121 may be disposed to be inclined in a direction toward the tissue carrier 110. For example, the magnetic field generator 121 may be formed to be inclined in the direction toward the surgical port 1221.

To this end, the magnetic field generation module 120 may further include a support 123 for supporting the magnetic field generator 121, and the number of supports 123 may correspond to the number of magnetic field generators 121.

The support 123 may be coupled to a biased position at one side of the magnetic field generator 121. Therefore, referring again to FIG. 5, the magnetic field generator 121 may be inclined such that one side to which the support 123 is coupled is positioned at a relatively higher level than the other side.

Since the magnetic field generator 121 is disposed to be inclined in this way, the plurality of magnetic field generators 121 may be inclined in the direction toward the surgical port 1221, and a magnetic field may be concentrated in the direction toward the surgical port 1221.

In addition, since the human eyeball is formed in an approximately spherical shape, the human eyeball includes a portion that protrudes convexly toward the outside of an eye. Therefore, in this way, when the magnetic field generator 121 is inclined in the direction toward the surgical port 1221 to concentrate a magnetic field, the tissue carrier 110 may be moved more precisely and efficiently.

The magnetic field generator 121 may include the coil, the core 1211, and a body 1212. As described above, the coil may be wound along the outer circumference surface of the core 1211 and may be a component through which a current supplied from a power source flows. Therefore, when a current flows through the coil, a magnetic flux is induced on the core 1211, and a magnetic field may be generated along the core 1211.

The body 1212 may be a component that constitutes a body of the magnetic field generator 121. The core 1211 may be disposed to pass through at least a portion of the body 1212. For example, at least a portion of the core 1211 may be disposed inside the body 1212. In addition, at least a portion of the core 1211 may be formed to extend outward from the body 1212. In this case, the portion of the core 1211 that extends outward from the body 1212 may be at a position close to the surgical port 1221.

Accordingly, a magnetic field generated from the magnetic field generator 121 may be generated to ultimately pass through a portion of the core 1211 exposed to the outside of the body 1212, that is, one end of the core 1211 close to the surgical port 1221, to be concentrated in the direction toward the surgical port 1221. Accordingly, the magnetic field generated from the magnetic field generator 121 may be concentrated in the direction toward the surgical port 1221.

As an alternative embodiment, the core 1211 may include at least one curved portion 1213 and an inclined portion 1214. For example, the core 1211 may include the curved portion 1213 bent at a certain angle in the direction toward the tissue carrier 110, and the inclined portion 1214 inclined in the direction toward the tissue carrier 110.

The curved portion 1213 may be a portion bent among portions of the core 1211. Specifically, the curved portion 1213 may be formed at the portion of the core 1211 exposed to the outside of the body 1212, and the core 1211 may be formed to be bent in the direction toward the surgical port 1221. Specifically, referring again to FIG. 6, the portion of the core 1211 exposed to the outside of the body 1212 may be bent in a downward direction of the curved portion 1213, that is, in the direction toward the surgical port 1221. Accordingly, when the magnetic field generation module 120 is positioned adjacent to an eyeball of a patient, the core 1211 may be bent at a certain angle in the direction toward the tissue carrier 110.

In addition, the plurality of magnetic field generators 121 may each include the core 1211, and each core 1211 may include the curved portion 1213.

Accordingly, a magnetic field generated from the plurality of magnetic field generators 121 may be more efficiently concentrated in the direction toward the surgical port 1221.

The inclined portion 1214 may be a portion formed to be inclined at an end portion among the portions of the core 1211. Specifically, the inclined portion 1214 may be formed at the end portion of the core 1211 and may be formed at an end portion of the portion exposed to the outside of the body 1212. In this case, the inclined portion 1214 may be formed to have a cross section similar to a hyperbola formed by obliquely cutting a cylinder. In this case, as shown in FIG. 6, the inclined portion 1214 may be formed to be inclined in the direction toward the surgical port 1221.

In addition, the plurality of magnetic field generators 121 may each include the core 1211, and each core 1211 may include the inclined portion 1214.

Accordingly, a magnetic field generated from the plurality of magnetic field generators 121 may be more efficiently concentrated in the direction toward the surgical port 1221.

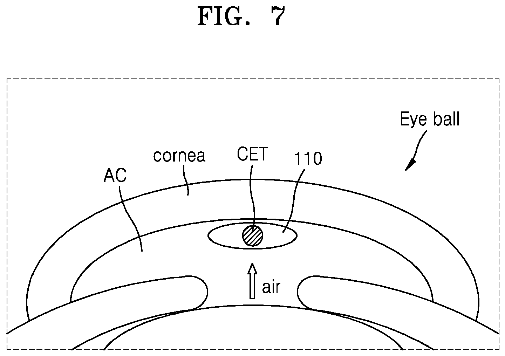

FIG. 7 is a schematic view illustrating a process of attaching CET to a cornea.

Referring to FIGS. 2 and 7, the corneal endothelial tissue transplantation system 100 according to an embodiment of the present disclosure may further include an air supply module 170.

The air supply module 170 may be a means for supplying air to the inserted tissue carrier 110 to move the CET attached to the tissue carrier 110 to be closer to a cornea.

Specifically, the air supply module 170 may supply air to the tissue carrier 110 to which the CET is attached, thereby moving the tissue carrier 110 upward in FIG. 7. Accordingly, the CET may attach to a corneal endothelium by moving upward together with the tissue carrier 110.

Thereafter, after the CET is attached to the cornea to a certain extent or more, a user may separate a wire attached to the tissue carrier 110 and may remove only the wire from an AC of an eyeball.

By including a configuration described above, according to the corneal endothelial tissue transplantation system 100 according to the present disclosure, there may be provided the corneal endothelial tissue transplantation system 100 capable of easily and precisely manipulating CET without inserting a separate tool into an AC of an eyeball.

In addition, according to the corneal endothelial tissue transplantation system 100 including such a configuration, a magnetic field may be efficiently concentrated in the direction toward the inserted tissue carrier 110, thereby improving the operation precision of a user.

In the specification (especially in the claims) of the embodiments, the use of the term "the" and similar indicating terms may correspond to both singular and plural. In addition, in the case where a range is described in the embodiment, since it includes the disclosure in which the individual values belonging to the range are applied, unless otherwise stated, it is the same as describing each individual value constituting the range in the detailed description. Finally, when there is no explicit or contradictory description of operations constituting the method according to the embodiment, the operations may be performed in a suitable order. The embodiments are not necessarily limited to the order in which the operations are described.

### [Industrial Applicability]

The present disclosure relates to a corneal endothelial tissue transplantation system for easily and precisely moving CET into an AC of an eyeball. In addition, embodiments of the present disclosure may be applied to a corneal endothelial tissue transplantation system used industrially.

## Claims

1. A corneal endothelial tissue transplantation system (100) comprising:
a tissue carrier (110) configured to be attached to cultured corneal endothelial tissue and configured to be inserted into an anterior chamber of an eyeball,
a magnetic field generation module (120) comprising a plurality of magnetic field generators (121) which generate a magnetic field for moving the tissue carrier (110), and a support plate (122) which supports the magnetic field generator (121);
a user input module (140) which receives an input signal for controlling the magnetic field generator (121) from a user,
wherein the support plate includes a surgical port (1221) formed through the support plate (122) configured to observe the inserted tissue carrier (110); and wherein the plurality of magnetic field generators (121) are disposed on one surface of the support plate (122) along a circumference of the surgical port (1221) such that the generated magnetic field is concentrated in a direction toward the surgical port,
wherein the tissue carrier comprises a wire (111) disposed along at least a portion of a circumference of the tissue carrier (110), and the wire includes a magnetic material.

2. The corneal endothelial tissue transplantation system of claim 1, wherein the tissue carrier is formed in a plate shape.

3. The corneal endothelial tissue transplantation system of claim 2, wherein the tissue carrier is inserted into the anterior chamber of the eyeball in a state in which at least a portion thereof is folded, and after the tissue carrier is inserted into the anterior chamber of the eyeball, the tissue carrier is unfolded by the magnetic field generation module.

4. The corneal endothelial tissue transplantation system of claim 1, wherein the magnetic field generator is disposed to be inclined in a direction toward the tissue carrier.

5. The corneal endothelial tissue transplantation system of claim 1, wherein the magnetic field generator comprises a core around which a coil is wound, wherein the core comprises a curved portion bent at a certain angle in a direction toward the tissue carrier.

6. The corneal endothelial tissue transplantation system of claim 1, further comprising an air supply module configured to supply air to inserted tissue carrier.

7. The corneal endothelial tissue transplantation system of claim 1, wherein the plurality of magnetic field generators comprises four magnetic field generators, and the magnetic field generators are disposed to form an angle of 90° around one point.

8. The corneal endothelial tissue transplantation system of claim 7, wherein the support plate is formed in a shape corresponding to the arrangement of the four magnetic field generators.

9. The corneal endothelial tissue transplantation system of claim 1, wherein the support plate has a plate shape extending in one direction and includes a flat portion (1222) for gripping by a user.

10. The corneal endothelial tissue transplantation system of claim 9,
wherein when a width direction of the support plate is defined as an xy plane,
a direction of a magnetic field applied to the tissue carrier is controlled in x- and y-axis directions by the user input module, and
the user controls the magnetic field in the z-axis direction by gripping the flat portion (1222) and moving the magnetic field generation module in a direction perpendicular to the xy plane of the support plate.

## Patentansprüche

1. Transplantationssystem (100) für Hornhautendothelgewebe, umfassend:
einen Gewebeträger (110), der dazu ausgelegt ist, an kultiviertem Hornhautendothelgewebe angebracht zu werden, und dazu ausgelegt ist, in eine vordere Kammer eines Augapfels eingeführt zu werden,
ein Magnetfelderzeugungsmodul (120), das eine Mehrzahl von Magnetfeldgeneratoren (121), die ein Magnetfeld zum Bewegen des Gewebeträgers (110) erzeugen, und eine Trägerplatte (122) umfasst, die den Magnetfeldgenerator (121) trägt;
ein Benutzereingabemodul (140), das ein Eingangssignal zum Steuern des Magnetfeldgenerators (121) von einem Benutzer empfängt,
wobei die Trägerplatte einen chirurgischen Port (1221) beinhaltet, der durch die Trägerplatte (122) ausgebildet und zur Beobachtung des eingeführten Gewebeträgers (110) ausgelegt ist; und wobei die Mehrzahl von Magnetfeldgeneratoren (121) auf einer Oberfläche der Trägerplatte (122) entlang eines Umfangs des chirurgischen Ports (1221) angeordnet ist, so dass das erzeugte Magnetfeld in einer Richtung zu dem chirurgischen Port konzentriert wird,
wobei der Gewebeträger einen Draht (111) umfasst, der entlang mindestens eines Abschnitts eines Umfangs des Gewebeträgers (110) angeordnet ist, und der Draht ein magnetisches Material beinhaltet.

2. Transplantationssystem für Hornhautendothelgewebe nach Anspruch 1, wobei der Gewebeträger in einer Plattenform ausgebildet ist.

3. Transplantationssystem für Hornhautendothelgewebe nach Anspruch 2, wobei der Gewebeträger in einem Zustand, in dem mindestens ein Teil davon gefaltet ist, in die vordere Kammer des Augapfels eingeführt wird und der Gewebeträger nach der Einführung des Gewebeträgers in die vordere Kammer des Augapfels durch das Magnetfelderzeugungsmodul entfaltet wird.

4. Transplantationssystem für Hornhautendothelgewebe nach Anspruch 1, wobei der Magnetfeldgenerator so angeordnet ist, dass er in einer Richtung zu dem Gewebeträger geneigt ist.

5. Transplantationssystem für Hornhautendothelgewebe nach Anspruch 1, wobei der Magnetfeldgenerator einen Kern umfasst, um den eine Spule gewickelt ist, wobei der Kern einen gekrümmten Abschnitt umfasst, der in einer Richtung zum Gewebeträger in einem bestimmten Winkel gebogen ist.

6. Transplantationssystem für Hornhautendothelgewebe nach Anspruch 1, ferner umfassend ein Luftzufuhrmodul, das dazu ausgelegt ist, dem eingeführten Gewebeträger Luft zuzuführen.

7. Transplantationssystem für Hornhautendothelgewebe nach Anspruch 1, wobei die Mehrzahl von Magnetfeldgeneratoren vier Magnetfeldgeneratoren umfasst und die Magnetfeldgeneratoren so angeordnet sind, dass sie einen Winkel von 90° um einen Punkt herum bilden.

8. Transplantationssystem für Hornhautendothelgewebe nach Anspruch 7, wobei die Trägerplatte in einer Form ausgebildet ist, die der Anordnung der vier Magnetfeldgeneratoren entspricht.

9. Transplantationssystem für Hornhautendothelgewebe nach Anspruch 1, wobei die Trägerplatte eine Plattenform aufweist, die sich in einer Richtung erstreckt und einen flachen Abschnitt (1222) zum Ergreifen durch einen Benutzer beinhaltet.

10. Transplantationssystem für Hornhautendothelgewebe nach Anspruch 9,
wobei, wenn eine Breitenrichtung der Trägerplatte als xy-Ebene definiert ist,
eine Richtung eines an den Gewebeträger angelegten Magnetfeldes durch das Benutzereingabemodul in x- und y-Achsrichtungen gesteuert wird und
der Benutzer das Magnetfeld in der z-Achsrichtung steuert, indem er den flachen Abschnitt (1222) ergreift und das Magnetfelderzeugungsmodul in einer Richtung senkrecht zu der xy-Ebene der Trägerplatte bewegt.

## Revendications

1. Système de transplantation de tissu endothélial cornéen (100) comprenant :
un support de tissu (110) configuré pour être fixé au tissu endothélial cornéen cultivé et configuré pour être inséré dans une chambre antérieure d'un globe oculaire, un module de génération de champ magnétique (120) comprenant une pluralité de générateurs de champ magnétique (121) qui génèrent un champ magnétique pour déplacer le support de tissu (110), et une plaque de support (122) qui supporte le générateur de champ magnétique (121) ;
un module d'entrée utilisateur (140) qui reçoit en provenance d'un utilisateur un signal d'entrée pour commander le générateur de champ magnétique (121),
dans lequel la plaque de support comporte un orifice chirurgical (1221) ménagé à travers la plaque de support (122) configuré pour observer le support de tissu (110) inséré ; et dans lequel la pluralité de générateurs de champ magnétique (121) est disposée sur une surface de la plaque de support (122) le long d'une circonférence de l'orifice chirurgical (1221) de telle sorte que le champ magnétique généré soit concentré dans une direction vers l'orifice chirurgical,
dans lequel le support de tissu comprend un fil (111) disposé le long d'au moins une partie de la circonférence du support de tissu (110), et le fil comporte un matériau magnétique.

2. Système de transplantation de tissu endothélial cornéen selon la revendication 1, dans lequel le support de tissu est en forme de plaque.

3. Système de transplantation de tissu endothélial cornéen selon la revendication 2, dans lequel le support de tissu est inséré dans la chambre antérieure du globe oculaire dans un état dans lequel au moins une partie de celui-ci est pliée, et après que le support de tissu a été inséré dans la chambre antérieure du globe oculaire, le support de tissu est déplié par le module de génération de champ magnétique.

4. Système de transplantation de tissu endothélial cornéen selon la revendication 1, dans lequel le générateur de champ magnétique est disposé de manière à être incliné dans une direction vers le support de tissu.

5. Système de transplantation de tissu endothélial cornéen selon la revendication 1, dans lequel le générateur de champ magnétique comprend un noyau autour duquel une bobine est enroulée, dans lequel le noyau comprend une partie incurvée courbée selon un certain angle dans une direction vers le support de tissu.

6. Système de transplantation de tissu endothélial cornéen selon la revendication 1, comprenant en outre un module d'alimentation en air configuré pour alimenter en air le support de tissu inséré.

7. Système de transplantation de tissu endothélial cornéen selon la revendication 1, dans lequel la pluralité de générateurs de champ magnétique comprend quatre générateurs de champ magnétique, et les générateurs de champ magnétique sont disposés pour former un angle de 90° autour d'un point.

8. Système de transplantation de tissu endothélial cornéen selon la revendication 7, dans lequel la plaque de support est formée selon une forme correspondant à l'agencement des quatre générateurs de champ magnétique.

9. Système de transplantation de tissu endothélial cornéen selon la revendication 1, dans lequel la plaque de support a une forme de plaque s'étendant dans une direction et comporte une partie plate (1222) destinée à être saisie par un utilisateur.

10. Système de transplantation de tissu endothélial cornéen selon la revendication 9,
dans lequel lorsqu'une direction de largeur de la plaque de support est définie en tant que plan xy,
une direction d'un champ magnétique appliqué au support de tissu est commandée dans les directions de l'axe x et de l'axe y par le module d'entrée utilisateur, et
l'utilisateur commande le champ magnétique dans la direction de l'axe z en saisissant la partie plate (1222) et en déplaçant le module de génération de champ magnétique dans une direction perpendiculaire au plan xy de la plaque de support.
